(19) Europäisches Patentamt
European Patent Office
Office européen des brevets

(11) Publication number: **0 136 033 A2**

# (12) EUROPEAN PATENT APPLICATION

(21) Application number: 84305559.1

(22) Date of filing: 15.08.84

(51) Int. Cl.⁴: A 61 K 47/00

(30) Priority: 22.08.83 AU 961/83

(43) Date of publication of application: 03.04.85 Bulletin 85/14

(84) Designated Contracting States: AT BE CH DE FR GB IT LI LU NL SE

(71) Applicant: ICI AUSTRALIA LIMITED, ICI House 1 Nicholson Street P.O.Box 4311, Melbourne Victoria 3001 (AU)

(72) Inventor: Evans, David Alwyn Charles, 32 Shields Street, Flemington, 3031 Victoria (AU)
Inventor: Hamilton, Robert John, 68 Forrester Street, Essendon 3040 Victoria (AU)
Inventor: McGarry, Errol James, 3 Argyll Court, Eltham North 3095 Victoria (AU)

(74) Representative: Atkinson, John David et al, Imperial Chemical Industries PLC Legal Department : Patents PO Box 6 Bessemer Road, Welwyn garden City Herts, AL7 1HD (GB)

## (54) Pour-on formulation for the control of parasites.

(57) The invention concerns a pour-on composition for topical application to animals to control endoparasites. The composition comprises an endoparasiticide and a carrier comprising at least one dialkyl ketone. The compositions show surprisingly improved efficacy and freedom from unacceptable skin reaction even when applied to sensitive breeds of animals.

In further embodiments the invention provides a process for the treatment of animals to control or eradicate endoparasites and a process for preparing endoparasiticidal compositions.

EP 0 136 033 A2

Z/PH.33206/EP

0136033

TITLE MODIFIED
see front page



## Pour-on Formulation for the Control of Parasites

This invention relates to compositions suitable for the topical application of chemicals to animals to control parasites. In particular it relates to compositions comprising systemically active chemicals for the control of parasites.

Oral and parenteral administration of parasiticides for the control of endoparasites are well known in the art and usually take the form of drenching or injecting animals with suitable formulations of the parasiticides. However, because of their inherent advantages, especially in ease of administration, recently much effort has been directed to the development of pour-on formulations. That is, formulations which when topically applied by pouring onto or spotting onto the surface of the animal, typically along the backline or spinal region, provide effective control of endoparasites. The pour-on method of administration has inherent advantages over oral and parenteral administration including, for example, ease of administration, removal of the need to prevent the animal from moving during administration, and easing of the requirement to maintain sterile formulations. Moreover, highly trained personnel are not required for topical

administration of parasiticide formulations.

One parasiticide which is particularly favoured for the control of endoparasites in animals is tetramisole (d,l-2,3,5,6-tetrahydro-6-phenylimidazo-[2,3-b]thiazole) and its laevo-rotatory optical isomer levamisole. Levamisole is now the more widely used product as it is twice as active as tetramisole and has twice the therapeutic ratio. In practice the inorganic salts of levamisole such as, for example, levamisole hydrochloride and levamisole dihydrogen phosphate, formulated in highly acidic aqueous solutions, have been used for drenching and injection. Tetramisole and levamisole have been described in US Patent Nos 3 274 209 and 3 463 786 respectively.

In view of the efficacy of levamisole in the control of endoparasites much of the work directed to the development of a pour-on formulation for the control of endoparasites has investigated the use of levamisole. Moreover, as the inorganic salts of levamisole which are used in drench and injectable compositions show negligible skin penetration the investigations have concentrated on the use of levamisole per se, that is levamisole base, and not on the use of inorganic salts of levamisole.

Pour-on compositions comprising tetramisole, levamisole and the acid-addition salts thereof have been developed which are effective in transporting the endoparasiticide through the skin of cattle. For example, Australian Patent Nos 489 412 and 490 027 disclose formulations in which the carriers include solvents such as spindle oil and isopropanol, aromatic hydrocarbons (eg xylene and benzene), ketones (eg cyclohexanone), and various alcohols. However, the carriers disclosed which are most effective in transporting the endoparasiticde through the skin may

also produce pain and severe tissue reactions such as swelling, dryness and cracking of the skin. Moreover, such compositions have been found to be generally ineffective as endoparasiticides when applied to sheep and to cause pain and severe skin reactions including severe damage to the hides and skin of the animals.

Australian Patent Application No 71 322/81 and its equivalent United States Patent 4 278 684 disclose the use of aliphatic carboxylic acids of pKa in the range of from 3 to 6 as skin penetration promoters to improve the efficacy of pour-on compositions comprising tetramisole or levamisole and a di(lower alkyl) dicarboxylic acid ester. The applicants have confirmed that the use of aliphatic carboxylic acids of pKa in the range of from 3 to 6 does enhance the uptake of the endoparasiticide in sheep but no improvement in uptake was noted in cattle. However, the use of alkanoic acids in pour-on compositions to promote the uptake of the endoparasiticide was found increase the level of adverse skin reactions in sheep, and especially in Merino and Merino-cross sheep which appear to be particularly susceptable to adverse skin reaction. Moreover, it was further found that the use of alkanoic acids in pour-on formulations comprising a hydroxylic solvent such as an alcohol (eg the compositions exemplified in United States Patent No 4 278 684) led to rapid decrease in the efficacy of the compositions on storage through degradation of the endoparasiticides levamisole and tetramisole.

Australian Patent Application No 81782/82 discloses a pour-on composition comprising tetramisole or levamisole in a carrier comprising at least 50% by weight of an alcohol ethoxylate. The application teaches that the use of an alcohol ethoxylate as a carrier, and especially at least 80% by weight of 2-(2-butoxyethoxy)ethanol as carrier, decreases the incidence of skin irritation and the specification exemplifies the use of such formulations in the treatment of cattle. The applicants have confirmed that such formulations are suitable for use in cattle. However, the applicants have found that such formulations are not suitable for use in sheep as they fail to give anthelmintically effective blood levels of the endoparasiticide, presumably through unsatisfactory transfer of the endoparasiticide through wool or wool grease cover, and their use results in unacceptable adverse skin reaction.

Australian Patent Application No 90 937/82 discloses the use of a mixture of a penetrating solvent and a bland solvent as a carrier to reduce or eliminate the incidence of adverse skin reactions in pour-on compositions comprising tetramisole or levamisole. In practice it has been found that such carriers, comprising an alcohol alkoxylate and a dialkyl ester of a dicarboxylic acid, dicarboxylate ester of a dihydric alcohol or a carboxylate ester of an alcohol alkoxylate, provide excellent pour-on compositions for the effective anthelmintic treatment of cattle and essentially overcome the problem of adverse skin reaction in sheep. However, under field conditions the compositions did not provide consistent anthelmintically effective blood levels of the endoparasiticide in all of the breeds of sheep tested.

While the prior-art discloses formulations which have provided progressively increasing endoparasiticidal efficacy and decreasing adverse skin reaction there remains a need in the art for an endoparasiticidal pour-on formulation with improved efficacy and decreased adverse skin reaction. In particular, there remains a need for an endoparasiticidally effective pour-on formulation which

may be applied to sensitive animals such as sheep without causing adverse skin reaction.

New endoparasiticidal pour-on formulations have now been found which, surprisingly, combine the features of improved efficacy, freedom from unacceptable skin reactions even when applied to sensitive breeds of animals, and long shelf life even when they incorporate levamisole or tetramisole which are relatively chemically sensitive and subject to decomposition.

Accordingly, the invention provides a composition for topical application to animals to control endoparasites which composition comprises an endoparasiticide and a carrier comprising at least one ketone selected from the group consisting of compounds of the formula I

$$R^1\text{-CO-}R^2 \qquad \text{I}$$

wherein $R^1$ and $R^2$, which may be the same or different, are selected from $C_1$ to $C_6$ alkyl and $C_5$ to $C_6$ cycloalkyl.

Endoparasiticides which may be used in the compositions of the present invention include: anthelmintics such as tetramisole, levamisole and avermectins including ivermectin; and flukicides such as triclabendazole [6-chloro-5-(2,3-dichlorophenoxy)-2-methylthiobenzimidazole] and rafoxanide { N-[3-chloro-4-(4-chlorophenoxy)phenyl]-2-hydroxy-3,5-diiodobenzamide } . Preferred endoparasiticides for use in the compositions of the present invention include tetramisole and levamisole, levamisole being more preferred because of its higher anthelmintic activity and therapeutic ratio.

Preferred ketones for use as carriers in the compositions of the present invention include compounds

of formula I in which at least one of the groups $R^1$ and $R^2$ is a branched chain alkyl group or a cycloalkyl group.

More preferred ketones for use as carriers in the compositions of the present invention include compounds of formula I wherein:

$R^1$ is selected from $C_1$ to $C_6$ alkyl; and

$R^2$ is selected from $C_4$ to $C_6$ alkyl.

Even more preferred ketones for use as carriers in the compositions of the present invention include compounds of formula I wherein:

$R^1$ is selected from the group consisting of methyl, ethyl, n-propyl, n-butyl, isobutyl and isoamyl; and

$R^2$ is selected from the group consisting of isobutyl and isoamyl.

Specific examples of preferred ketones for use as carriers in the compositons of the present invention include methyl isobutyl ketone and di-(isobutyl) ketone.

The carriers used in the compositons of the present invention may comprise, in addition to at least one ketone of formula I as hereinbefore defined, one or more organic solvents. Suitable additional organic solvents include: dialkyl esters of dicarboxylic acids such as, for example, di($C_1$ to $C_6$ alkyl)esters of $C_2$ to $C_6$ dicarboxylic acids; di(carboxyl)esters of alkylene glycols such as, for example, di($C_2$ to $C_6$ carboxyl)

esters of alkylene glycols such as, for example, di($C_2$ to $C_6$ carboxyl) esters of ethylene glycol, diethylene glycol, triethylene glycol, propylene glycol and butan-1,4-diol; tri($C_2$ to $C_6$ carboxyl) esters of glycerol; and mono- alkyl ethers of alkylene glycols such as, for example, the products of reaction of one mole of a $C_1$ to $C_6$ alcohol with from one to three moles of an alkylene oxide selected from ethylene oxide, propylene oxide and mixtures thereof. However, preferably the optional, additional organic solvent is a non-hydroxylic solvent.

Preferred optional, additional organic solvents include the di($C_2$ to $C_6$ carboxyl) esters of ethylene glycol, diethylene glycol, triethylene glycol, propylene glycol and butan-1,4-diol. Specific examples of the preferred optional, additional organic solvents include ethylene glycol diacetate, diethylene glycol diacetate and butan-1,4-diol diacetate.

When an optional, additional organic solvent is employed in the compositions of the present invention preferably the weight ratio of the ketone of the composition of the present invention to the optional, additional carrier is in the range of from 9:1 to 1:2.

The compositions of the present invention optionally may also comprise one or more aliphatic carboxylic acids. In general it has been found that the compositions of the present invention do not require the addition of carboxylic acids to promote uptake of the endoparasiticide. However, in some carriers comprising tetramisole or levamisole as endoparasiticide the presence of an aliphatic carboxylic acid may promote the uptake of the endoparasiticide. When used in tetramisole or levamisole compositions of the present invention preferably the molar ratio of the aliphatic carboxylic acid to the tetramisole or levamisole does not exceed 1.1 and more preferably it

is in the range of 0.5 to 1.0. Preferred aliphatic carboxylic acids for use in tetramisole or levamisole containing compositions of the present invention are the $C_1$ to $C_8$ alkanoic acids, more preferably $C_1$ to $C_4$ alkanoic acids, and especially acetic acid and propionic acid.

In compositions of the present invention comprising tetramisole or levamisole as endoparasiticide and a hydroxylic solvent as an additional organic solvent, preferably aliphatic carboxylic acids are not added in order to attempt to promote the uptake of the endoparasiticde. Surprisingly, it has been found that the endoparasiticides tetramisole and levamisole undergo relatively rapid degradation in the presence of hydroxylic solvents and aliphatic carboxylic acids and as a result such compositions have an unsatisfactory shelf life. Therefore, as hereinbefore indicated, preferably any optional, additional organic solvent incorporated into the compositions of the present invention is a non-hydroxylic solvent.

The compositions of the present invention may optionally contain additives to stabilize, preserve, colour or otherwise improve the storage properties and/or ease of application of the compositions. Examples of stabilizers which may find application in the compositions of the present invention are antioxidants such as, for example, phenol antioxidants. Included among the preferred antioxidants is the phenol antioxidant 2,6-di(t-butyl)-4-methylphenol. When used in the compositions of the present invention the antioxidant or mixture of antioxidants is preferably used at a concentration in the range of from 0.01 to 1.0 percent w/v, more preferably 0.05 to 0.1 percent w/v of the final composition.

It will be evident to those skilled in the art

that in certain circumstances it may be desirable for the compositions of the invention to contain a colouring agent or dye. For example, the presence of a colouring agent in the compositions of the invention may provide a quick, effective way for identifying the compositions per se. Moreover, animals treated by topical application of the compositions may be readily identified thereby allowing unnecessary and undesirable duplicate treatment to be avoided. When used in the compositions of the present invention preferably the colouring agent or dye is non-fast and/or watersoluble so that the colouring agent or dye will fade or be washed away within a suitable period without deleterious effect to the skin or wool. Colouring agents or dyes which have been found to be useful in the compositions of the present invention include edicol erythrosine and rhodamine B. When used in the compositions of the present invention preferably the colouring agent or dye is used at a concentration in the range of from 0.001 to 0.05 percent w/v of the final composition.

Further optional additives which will be evident to those skilled in the art include, for example, bitter tasting non-toxic agents to deter the animals from licking the compositions off their hide or skin or the hides or skins of other animals.

The compositions of the present invention may include, in addition to one of more endoparasiticides, one or more agents which, when topically applied to animals, are effective in treating diseases of animals or generally improving the health and/or well being of animals. Particularly useful agents which may be incorporated into the compositions of the invention are ectoparasiticides which are useful in the control or eradication of animal ectoparasites. Examples of preferred ectoparasiticides which may be used in the compositions of the invention are synthetic pyrethroids such as, for example, permethrin, cypermethrin, deltamethrin, phenothrin, cyphenothrin, flumethrin, cyfluthrin and cyhalothrin.

When a synthetic pyrethroid is used as an ectoparasiticide in the compositions of the present invention the concentration of the pyrethroid in the formulation will depend to a large extent on the pyrethroid used, the ectoparasite to be controlled and the animal to be treated. The specific concentration may be readily determined by those skilled in the art so that topical application of the quantity of the composition required to give effective control of endoparasites will also give effective control of ectoparasites. Typically, the concentration of the present invention will be in the range of from 0.2 to 5.0 percent w/v of the final composition.

The invention also provides in a further embodiment a process for the control or eradication of endoparasites in an animal which process comprises topically applying to said animal an endoparasiticidally effective amount of a composition as hereinbefore defined.

In the method of treatment according to the invention, the compositions of the invention are topically applied to the animal generally by pouring onto or spotting onto the surface of the animal along the backline or spinal region. The compositions may also be applied by brushing or rolling onto the surface of the animal but, for convenience, the compositions are preferably applied by simply pouring onto the back of the animal.

The compositions of the present invention may be used in the treatment of endoparasite infestations in a range of animals and especially warm-blooded animals including mammals. The compositions may be

used in the treatment of domestic or farm animals such as, for example, cattle, sheep, pigs, horses, goats, dogs and cats, and find particular utility in the treatment of endoparasite infestations in cattle and sheep.

As hereinbefore indicated, endoparasiticides which may be used in the compositions of the present invention include: anthelmintics such as tetramisole, levamisole and avermectins including ivermectin; and flukicides such as triclabendazole and rafoxanide. The concentration of the endoparasiticide used in the compositions of the invention will depend to a large extent on the specific endoparasiticide to be used, the animal to be treated, and the amount of composition to be applied. The specific concentration may be readily determined by those skilled in the art so that when the selected quantity of the composition of the endoparasiticide is topically applied to an animal of known bodyweight then the endoparasiticide will be applied in an amount sufficient to effectively control the infestation of endoparasites in the animal. Typically, in the compositions of the present invention, the concentrations of the endoparasiticides would be in the following ranges: tetramisole or levamisole from 1 to 30% w/v; ivermectin from 0.01 to 2.0% w/v; triclabendazole from 0.5 to 15.0% w/v; and rafoxanide from 0.5 to 15.0% w/v. Typically the amount of composition of the present invention applied to the animal to be treated is sufficient to give dose rates of the endoparasiticides in the following dose ranges: levamisole from 10 to 25 mg/kg; tetramisole from 15 to 50 mg/kg; ivermectin from 0.2 to 1.0 mg/kg; triclabendazole from 2 to 10 mg/kg; and rafoxanide from 10 to 20 mg/kg.

As hereinbefore indicated the preferred endoparasiticide for use in the compositions of the present invention is levamisole. Levamisole is a

particularly effective endoparasiticide against a range of gastro intestinal and pulmonary nematodes such as, for example, Haemonchus spp., Ostertagia spp., Trichostrongylus spp., Chabertia spp. and Dictyocaulus spp. Preferably levamisole is applied at a dose rate of approximately 20 mg/kg of animal bodyweight. Therefore, a composition containing, for example, 10% w/v of levamisole would preferably be applied at a rate of 0.2 ml/kg whereas a composition containing 20% w/v of levamisole would preferably be applied at a rate of 0.1 ml/kg.

The compositions of the present invention provide a significant advance over prior-art compositions as they combine the features of improved efficacy (readily demonstrated by the higher endoparasiticide blood levels achieved by the compositions of the present invention), freedom from unacceptable adverse skin reaction, and long shelf life. The higher endoparasiticide blood levels obtained by topical application of the compositions of the present invention is both unexpected and significant. For example, when compositions of the present invention and compositions of Australian Patent Application No 90937/82 were compared , the compositions of the present invention gave levamisole blood levels of the order of 3 to 10 times higher in sheep. Moreover, no unacceptable adverse skin reaction was noted the sheep.

The compositions of the present invention may be prepared by dissolving the endoparasiticide, any additional topically effective agent for treating diseases of animals or generally improving the health and/or well being of animals, and any additive to improve the storage properties and/or ease of application of the compositions, in a carrier as hereinbefore defined. Accordingly in a further aspect the invention provides a process for the preparation of a composition for topical application to animals to control endoparasites which process comprises dissolving said endoparasiticide in a carrier comprising at least one ketone of formula I as hereinbefore defined.

Typically, the compositions of the present invention are prepared by dissolving the endoparasiticide in the carrier at ambient temperature. However, lower or higher temperatures may be utilized if required, temperatures in the range of from 0 to 100°C being preferred and temperatures in the range of from 15 to 40°C being more preferred. The mixtures of ingredients may be agitated by, for example, shaking or stirring in order to aid dissolution of the endoparasiticide in the carrier.

The invention is now illustrated by but not limited to the following examples in which all parts and percentages are by weight unless otherwise specified.

Examples 1 to 8

These Examples illustrate the preparation of compositions of the present invention.

The compositions were prepared by mixing, at room temperature, the components in the proportions indicated in Table 1 and stirring the mixture until a homogeneous solution was obtained.

TABLE 1

| Component | Component Concentration (parts by weight) Example No | | | | | | | |
|---|---|---|---|---|---|---|---|---|
| | 1 | 2 | 3 | 4 | 5 | 6 | 7 | 8 |
| L | 9.90 | 10.42 | 10.10 | | 10.60 | 9.90 | - | - |
| R | - | - | - | 5.40 | - | - | - | - |
| I | - | - | - | - | - | - | 0.50 | - |
| T | - | - | - | - | - | - | - | 5.40 |
| C | - | - | - | - | - | 0.99 | - | - |
| MIBK | 34.16 | 50.57 | - | 54.42 | 53.16 | 33.67 | 38.63 | 54.42 |
| DIBK | - | - | 40.67 | - | - | - | - | - |
| EGDA | 55.84 | 35.25 | 45.58 | - | - | 55.43 | 60.86 | 40.17 |
| DEGDA | - | - | - | 40.17 | - | - | - | - |
| BGDA | - | - | - | - | 32.42 | - | - | - |
| EE | - | 0.01 | 0.01 | 0.01 | 0.01 | - | - | 0.01 |
| RB | 0.10 | - | - | - | - | 0.01 | 0.01 | - |
| PA | - | 3.75 | 3.64 | - | 3.81 | - | - | - |

Code for TABLE 1

| | | |
|---|---|---|
| L | - | Levamisole |
| R | - | Rafoxanide |
| I | - | Ivermectin |
| T | - | Triclabendazole |
| C | - | Cyhalothrin |
| | | |
| MIBK | - | Methyl isobutyl ketone |
| DIBK | - | Di(isobutyl) ketone |
| | | |
| EGDA | - | Ethylene glycol diacetate |
| DEGDA | - | Diethylene glycol diacetate |
| BGDA | - | Butan-1,4-diol diacetate |
| | | |
| EE | - | Edicol erythrosine |
| RB | - | Rhodamine B |
| | | |
| PA | - | Propionic Acid |

Examples 9 to 11

These Examples illustrate the efficacy of compositions of the present invention in producing surprisingly high and anthelmintically effective blood levels of levamisole in sheep.

The test animals were treated with a composition of the invention by pour-on application along the spine. Details of the animals treated, dose rate and composition of the invention used are given in Table 2.

TABLE 2

| Example No | Test Animal | Dose Rate (mg/kg) | Test Composition (Example No) |
|---|---|---|---|
| 9 | Merino sheep | 10 | 1 |
| 10 | Merino sheep | 20 | 2 |
| 11 | Merino sheep | 20 | 3 |

Blood samples were drawn from the animal at regular intervals and the resulting serum was analyzed for levamisole by a procedure involving clean-up on "Bond Elut" $C_{18}$ columns ("Bond Elut" is a registered trade mark) followed by a final determination by high performance liquid chromatography.

The results are shown in Tables 3 to 5 and illustrate that blood levamisole levels of 0.3 $\mu$ g/cm$^3$ and higher are rapidly reached and maintained over a period of seven hours when compositions of the invention are topically applied at levamisole dose rates of 20 mg/kg animal body weight. A blood levamisole level of 0.3 $\mu$ g/cm$^3$ or above is known in the art to give effective control of helminth endoparasites. No unacceptable adverse skin reactions were noted during or after the application of any of the compositions.

TABLE 3 - Example 9

| Animal No | Levamisole conc. ( μ g/cm$^3$) in Serum | | | | |
|---|---|---|---|---|---|
| | Hours: 1 | 2 | 3 | 5 | 7 |
| 1 | 0.38 | 0.36 | 0.30 | 0.31 | 0.30 |
| 2 | 0.21 | 0.29 | 0.23 | 0.24 | 0.21 |
| 3 | 0.13 | 0.16 | 0.16 | 0.19 | 0.16 |
| 4 | 0.08 | 0.09 | 0.09 | 0.18 | 0.14 |
| 5 | 0.45 | 0.30 | 0.27 | 0.20 | 0.12 |
| Mean | 0.25 | 0.30 | 0.21 | 0.22 | 0.19 |

TABLE 4 - Example 10

| Animal No | Levamisole conc. ( µ g/cm$^3$) in Serum | | | | |
|---|---|---|---|---|---|
| Hours: | 1 | 2 | 3 | 5 | 7 |
| 1 | 1.85 | 1.58 | 1.50 | 1.14 | 0.75 |
| 2 | 0.26 | 0.90 | 1.02 | 0.72 | 0.82 |
| 3 | 0.70 | 0.40 | 0.31 | 1.25 | 1.27 |
| 4 | 1.03 | 0.95 | 1.19 | 0.84 | 0.97 |
| 5 | 0.36 | 0.37 | 0.45 | 0.38 | 0.34 |
| 6 | 1.92 | 1.77 | 2.11 | 1.65 | 1.21 |
| 7 | 0.67 | 0.60 | 0.73 | 0.50 | 0.62 |
| 8 | 0.29 | 0.29 | 0.54 | 0.55 | 0.51 |
| Mean | 0.89 | 0.86 | 0.98 | 0.88 | 0.81 |

TABLE 5 - Example 11

| Animal No | Levamisole conc. ( µ g/$cm^3$) in Serum Hours: 1 | 2 | 3 | 5 | 7 |
|---|---|---|---|---|---|
| 1 | 2.44 | 2.24 | 2.25 | 0.97 | 0.46 |
| 2 | 0.57 | 0.82 | 0.70 | 0.45 | 0.20 |
| 3 | 0.63 | 0.79 | 0.63 | 0.28 | 0.14 |
| 4 | 0.50 | 0.99 | 0.82 | 0.54 | 0.41 |
| Mean | 0.99 | 1.16 | 1.08 | 0.56 | 0.30 |

Comparative Example A

This Example illustrates the inability of a prior-art composition to produce anthelmintically effective levamisole blood levels in sheep.

Each of the animals in a group of five Merino wethers were treated with the following prior art composition at a dose rate of 20 mg/kg following the procedure described for Examples 9 to 11.

Comparative Example A

| Component | Concentration (parts by weight) |
|---|---|
| Levamisole | 9.80 |
| 2-(n-Butoxy)ethanol | 56.39 |
| Ethylene Glycol Diacetate | 33.80 |
| Edicol Erythrosine | 0.01 |
| Density | 1.02 g/$cm^3$ |

The levamisole blood levels in each of the sheep were measured following the procedure described for Examples 9 to 11 and the results are shown in Table 6 below. No unacceptable adverse skin reactions were noted during or after the application of the composition.

The results clearly illustrate that the prior art composition gives a significantly lower blood levamisole level in sheep (of the order of 3 to 10 fold lower) than provided by compositions of the present invention applied at the same dose rate (Examples 10 and 11; Tables 4 and 5. Moreover, the blood levamisole level provided by the compositions of the present invention was well in excess of the desired level of 0.3 μ g/$cm^3$ which is generally recognized in the art to give effective control of helminth endoparasites, whereas the blood levamisole level provided by the prior art composition is below this desired level.

TABLE 6 - Comparative Example A

| Animal No | Levamisole conc. ( μ g/cm$^3$) in Serum | | | | |
|---|---|---|---|---|---|
| | Hours: 1 | 2 | 3 | 5 | 7 |
| 6 | 0.06 | 0.07 | 0.11 | 0.11 | 0.12 |
| 7 | 0.04 | 0.11 | 1.19 | 0.25 | 0.33 |
| 8 | 0.00 | 0.05 | 0.07 | 0.07 | 0.06 |
| 9 | 0.06 | 0.15 | 0.16 | 0.25 | 0.28 |
| 10 | 0.06 | 0.22 | 0.35 | 0.35 | 0.44 |
| Mean | 0.04 | 0.12 | 0.18 | 0.21 | 0.25 |

Example 12

This Example illustrates the freedom from unacceptable adverse skin reaction observed with compositions of the present invention in comparison to prior-art compositions comprising a hydroxylic solvent.

A group of six Merino sheep were selected, two were treated with a composition comprising 10% w/v levamisole in 2-(n-butoxy)ethanol (Comparative Example B) and four were treated with the composition of Example No 1, following the same procedure as that described for Examples 9 to 11. The animals were examined for signs of skin irritancy 14 days, and then again 28 days, after treatment.

The results are shown in Table 7 and clearly

demonstrate that when compositions of the present invention are topically applied to sensitive animals such as sheep, no unacceptable adverse skin reaction occurs. Whereas in contrast, topical application of prior-art compositions comprising a hydroxylic solvent as carrier results in unacceptable adverse skin reaction which in turn reduces the quality and value of the animals fleece.

TABLE 7

| Composition | Sheep No | Skin Irritancy | |
|---|---|---|---|
| | | At 14 days | At 28 days |
| Example 1 | 1 | None | None |
| " " | 2 | None | None |
| " " | 3 | None | None |
| " " | 4 | None | None |
| Comparative Example B | 5 | Hard scab in fleece | Hard scab in fleece and break in wool |
| Comparative Example B | 6 | Hard scab in fleece | Hard scab in fleece and break in wool |

CLAIMS

1. A composition for topical application to animals to control endoparasites which composition comprises an endoparasiticide and a carrier comprising at least one ketone selected from the group of compounds of the formula I:

$$R^1\text{-CO-}R^2 \qquad I$$

wherein $R^1$ and $R^3$, which may be the same or different, are selected from $C_1$ to $C_6$ alkyl and $C_5$ and $C_6$ cycloalkyl.

2. A composition according to claim 1 wherein in the ketone of formula I $R^1$ is selected from the group consisting of propyl, n-butyl, isobutyl and isoamyl and $R^2$ is selected from the group consisting of isobutyl and isoamyl.

3. A composition according to any one of claims 1 and 2 wherein said ketone is selected from methyl isobutyl ketone and di(isobutyl) ketone.

4. A composition according to any one of claims 1 to 3 inclusive wherein said carrier additionally comprises an organic solvent selected from the group consisting of: di($C_1$ to $C_6$ alkyl) esters of $C_2$ to $C_6$ dicarboxylic acids; and the di($C_2$ to $C_6$ carboxyl) esters of ethylene glycol, diethylene glycol, triethylene glycol, propylene glycol and butan-1,4-diol.

5. A composition according to any one of claims 1 to 4 inclusive wherein said carrier additionally comprises an organic solvent selected from ethylene glycol diacetate, diethylene glycol diacetate and butan-1,4-diol acetate.

6. A composition according to either of claims 4 and 5 wherein in the carrier the weight ratio of said ester to said additional organic solvent is in the range of from 9:1 to 1:2.

7. A composition according to any one of claims 1 to 6 inclusive wherein said endoparasiticide is selected from the group consisting of: tetramisole, levamisole, the avermectins, triclabendazole, and rafoxanide.

8. A composition according to any one of claims 1 to 7 inclusive wherein said endoparasiticide is levamisole, and is present in a concentration in the range of from 1.0 to 30% w/v.

9. A composition according to any one of claims 1 to 8 inclusive wherein said composition additionally comprises one or more aliphatic carboxylic acids, and in which the mole ratio of said aliphatic carboxylic acid to levamisole is in the range of from 0.5:1 to 1.1:1, and said composition does not contain a hydroxylic solvent.

10. A composition according to any one of claims 1 to 9 inclusive wherein said composition additionally comprises an ectoparasiticide selected from the synthetic pyrethroids permethrin, cypermethrin, deltamethrin, phenothrin, cyphenothrin, flumethrin, cyfluthrin and cyhalothrin.

DCF0212PC009
GC16 - 8 Aug 84